Europäisches Patentamt

European Patent Office

Office européen des brevets

(11) Publication number: **0 341 300 B1**

(12) **EUROPEAN PATENT SPECIFICATION**

(45) Date of publication of patent specification: **09.03.94**   (51) Int. Cl.⁵: **C12N 5/02**, A61K 31/365

(21) Application number: **89900474.1**

(22) Date of filing: **16.11.88**

(86) International application number:
**PCT/US88/03957**

(87) International publication number:
**WO 89/05346 (15.06.89 89/13)**

(54) **STIMULATION OF STEM CELL GROWTH BY THE BRYOSTATINS.**

(30) Priority: **23.11.87 US 123736**

(43) Date of publication of application:
**15.11.89 Bulletin 89/46**

(45) Publication of the grant of the patent:
**09.03.94 Bulletin 94/10**

(84) Designated Contracting States:
**AT BE CH DE FR GB IT LI LU NL SE**

(56) References cited:

**Biochemical and Biophysical Research Communications, (Duluth, Minnesota, USA), vol. 131, Issued 30 Sept. 1985, Berkow et al, "Bryostatin, A non-phorbol macrocyclic lactone, activates intact human polymorphonuclear leukocytes and binds to the phorbol ester receptor", pages 1109-1116, entire document**

(73) Proprietor: **THE JOHNS HOPKINS UNIVERSITY 34th and Charles Streets Baltimore, Maryland 21218(US)**

(72) Inventor: **MAY, Stratford, W. 906 Kingston Road Baltimore, MD 21212(US)**
Inventor: **SENSENBRENNER, Lyle, Lee 1035 Devonshire Road Grosse Point Park, MI 48230(US)**

(74) Representative: **Dean, John Paul et al Withers & Rogers 4 Dyer's Buildings Holborn London EC1N 2JT (GB)**

Clinical Research, (Thoroface, New Jersey, USA), vol. 33, Issued 1985, Berkow et al, "Bryostatin activates human neutrophils by binding to the phorbol receptor", page 335A

Blood (Orlando, Florida, USA), vol. 68, Issued Nov. 1986, May et al, Bryostatin replaces GM-CSF by directly stimulating normal CFU-GM", page 147a abstract no. 466.

Proceedings of the National Academy Science, (Washington, D.C., USA), Vol. 84, issued December 1987, May et al, "Anti-neoplastic bryostatins are multipotential stimulators of human hematopoietic progenitor cells", pages 8483-8487.Entire document.

Blood (Orlando Florida) Vol. 70, issued November 1987, Gebbia et al, "Bryostatin-I stmulates multipotential murine hematopoietic stem cells in vivo",page 153a, Abstract no. 466.

**Description**

The work reported here was partially supported by grants from National Institutes of Health. The U.S. Government may retain certain rights in this invention.

## TECHNICAL FIELD OF THE INVENTION

This invention relates to chemical agents having stimulatory effects on growth and activity of hematopoietic cells. More particularly, the invention relates to the use of bryostatins to stimulate growth and activity of hematopoietic cells.

## BACKGROUND OF THE INVENTION

The bryostatins are a family of macrocyclic lactones which have been isolated from specimens of marine bryozoans. The sea-mat Bugula neritina has been the source of many of the bryostatins. Others have been isolated from the marine animal Amathia convoluta, also a member of the phylum Bryozoa.

All of the bryostatins are derived from the bryopyran ring system, (Pettit et al, Journal of Natural Products, Vol. 46, pp. 528-531, 1983) and all are reported to have antineoplastic activity against a single murine leukemia cell line, the P388 lymphocytic leukemia line. The bryostatins substantially inhibit P388 cell growth in vitro, and also are able to increase the life span of mice injected with P388 lymphocytic leukemia cells. Bryostatins 1-8 are described in Pettit U.S. 4,560,774 and U.S. 4,611,066.

There are many health conditions which result in a deficiency of hematopoietic cells. For example, a problem often arises in the treatment of cancers with antineoplastic agents; these agents can have an inhibitory effect on normal bone marrow progenitor cells (stem cells). In addition, there are many diseases in which neutropenias result, such as acquired immune deficiency syndrome (AIDS).

There are also many conditions in which erythroid cells are insufficient to support normal activity, such as congenital anemias, and Fanconi's anemia. Diseases such as rheumatoid arthritis and lupus erythematosus, often result in depletion of certain populations of blood cells. Therefore there is a continuing need in the art for agents which are safe to administer and which stimulate the development of various types of haematopoietic cells.

## SUMMARY OF THE INVENTION

It is an object of the present invention to provide a method of stimulating stem cells to proliferate and to differentiate to form erythrocytes, in vitro.

It is yet another object of the present invention to provide a method of activating the cytotoxicity of neutrophils and other immune regulatory cells, e.g T lymphocytes and macrophages, in vitro.

In one embodiment of the invention there is provided a method of stimulating stem cells to form erythrocytes comprising: obtaining a cell preparation containing stem cells; and incubating the cell preparation in vitro with a bryostatin having an acylated C20 carbon in an amount effective to stimulate formation of erythrocytes, whereby said formation is stimulated.

In yet another embodiment of the present invention there is provided the use of a bryostatin having an acylated C20 carbon in the preparation of a composition for the stimulation of formation of erythrocytes.

In yet another embodiment of the present invention, there is provided a method of activating the cytotoxicity of neutrophils and other immune regulatory cells, comprising: obtaining a cell preparation containing neutrophils or other immune regulatory cells; and incubating the cell preparation in vitro with an amount of a bryostatin having an acylated C20 carbon effective to activate the neutrophils or other immune regulatory cells, whereby said cells' cytotoxicity is activated.

In another embodiment of the present invention there is provided the use of a bryostatin having a acylated C20 carbon in the preparation of a composition for the activation of neutrophil or other immune regulatory cell cytotoxicity.

The methods of the present invention provide means of stimulating stem cell growth with molecules which show low levels of toxicity. The bryostatin molecules, which are based on a 37 carbon macrocyclic lactone structure, can substitute for naturally occurring colony stimulating factors for some purposes. However, in other ways the activity of the bryostatins appears to differ from that of naturally occurring colony stimulating factors. Thus the bryostatins, but not colony stimulating factors, stimulate stem cell differentiation without also stimulating growth of neoplastic cells.

3

## BRIEF DESCRIPTION OF THE DRAWINGS

Figure 1 depicts the chemical structures of the bryostatins. Panel A: bryostatin 1; panel B: bryostatin 9; panel C: bryostatin 8; panel D: bryostatin 3; panel E: bryostatin 13.

Figure 2 shows the effect of bryostatin on granulocyte-macrophage colony growth in vitro.

Figure 3 shows the effect of bryostatin 1 on the growth of transferred clones. Clones were initially grown in the presence of bryostatin 1, and transferred to media containing either bryostatin 1, recombinant human GM-CSF, or media alone.

Figure 4 depicts the effect of bryostatin 1 on growth of human erythroid progenitors.

Figure 5 shows the generation of chemiluminescence by neutrophils incubated with bryostatins 1 and 13, as well as recombinant human GM-CSF, and phorbol 12-myristate 13-acetate (TPA).

## DETAILED DESCRIPTION

It is a finding of the present inventors that certain bryostatins, are able to effect hematopoiesis. The morphological analysis of the colonies which are stimulated by bryostatins reveals that primarily mixed granulocyte-macrophage colonies and pure granulocyte colonies are formed from light-density, adherence-depleted, normal human bone marrow cells.

The bryostatins themselves can be obtained by extraction and purification from the marine bryozoan Bugula neritina. Description of the extraction and purification techniques can be found in Pettit et al, Journal of American Chemical Society, Vol. 104, pp. 6846, 1982, and Pettit et al, Journal of Natural Products, Vol. 46, pp. 528-531, 1983. Some of these compounds are also described in U.S Patents 4,560,774 and U.S 4,611,066. Generally, the bryostatins can be isolated and purified by solvent extraction, partition chromatography, silica gel chromatography, liquid-liquid distribution in a Craig apparatus, absorption on resins and crystallization from solvents. Isolation and purification methods can be monitored at each step by performing in vitro or in vivo anti-tumour tests, or by looking for activation of neutrophils or stimulation of formation of granulocytes, macrophages or erythrocytes from stem cells.

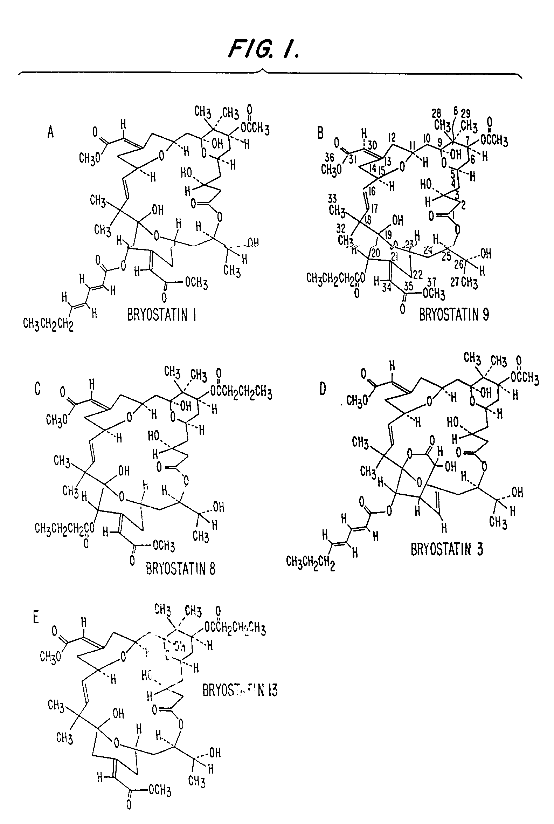

Not all bryostatins have the same amount of activity. For example, in the granulocyte-macrophage colony growth assay, bryostatin 1 has more activity than 3 which had more activity than 8 which had more activity than 9. Bryostatin 13 was totally inactive in this assay. When the structure-activity relationships of the bryostatins are examined, the results indicate that both the hematopoietic colony stimulating activity as well as the functional activation of neutrophils may be dependent upon acylation of carbon 20 (see figure 1). Thus, when the short-chain, saturated butyric acid was substituted in bryostatin 1 at carbon 20, the resulting bryostatin 9 was a much less potent stimulator of granulocyte-macrophage colony forming units (GM-CFU). This decrease in activity was restored when a substitution involving a longer acyl group at carbon 7 was made, as in bryostatin 8. However, when the acyl substitution on carbon 20 was completely removed, as in bryostatin 13, both colony stimulating activity and neutrophil activation was lost. Furthermore, the loss of activity does not result from a toxic effect of bryostatin 13, since when bryostatin 1 was mixed with bryostatin 13, the expected degree of granulocyte-macrophage colony formation obtained with broystatin 1 alone was observed. Bryostatin 13, which is inactive in the hematopoietic stimulating activities reported herein, is reported by others to retain potent antineoplastic activity. (See Pettit, et al, Journal of Organic Chemistry, (1957).)

Bryostatins 1, 3, 8 and 9 (see Figure 1) have been found to mediate a dose dependent sitmulation of granulocyte and macrophage colony forming units (CFU-GM). The maximum effective concentration is between about 1 and 100 nM. Bryostatins 1 and 8 are the most potent of the four active congeners tested. They show colony stimulating activity at concentrations as low as 0.01 nM. Bryostatin 3 is less potent than broystatins 1 and 8 having a maximal response between about 10 and 100 nM. Bryostatin 9 is a weaker stimulator of normal colony growth, and bryostatin 13 is completely inactive. Thus the hierarchy of bryostatin activity is that bryostatin 1 is most active, and bryostatins 3, 8 and 9 are increasingly less active, and bryostatin 13 is totally inactive.

Cell preparations containing stem cells can be obtained from bone marrow or from peripheral blood cells. These can be purified using techniques known in the art, such as density gradient centrifugation, to remove red blood cells. Alternatively, stem cells can be purified using antibodies which are specific for stem cell antigens. (See, Civin et al, Journal of Immunology, Vol. 133, p. 157, 1984.)

Amounts of bryostatin which are effective for stimulating hematopoiesis or neutrophil and other immune regulatory cell activation, can be determined using any of the assays known in the art. For example, early and late erythrocyte progenitors can be enumerated using the plasma clot method described in McLeod et al, Blood, Vol. 44, p. 517, 1974. Stimulation of such erythrocyte progenitors requires the presence of

4

erythropoietin. For bryostatin 1, optimum stimulation of erythrocytes was seen at about $10^{-12}$M to about $10^{-10}$M.

For determining effective concentrations of bryostatins for granulocyte and macrophage colony formation, light density, adherence-depleted human bone marrow cells can be obtained. The cells can be suspended in a suitable medium, such as supplemented McCoy's 5A medium containing 0.3% agar and plated in 35 mm petri dishes as described in Pike et al, Journal of Cell Physiology, Vol. 76, pp. 77-83, 1970. For the bryostatins which have been tested and found to be effective, i.e., 1, 3, 8 and 9, maximum stimulatory concentrations are between about $10^{-7}$M and $10^{-10}$M.

To determine the effective amount of bryostatin for neutrophil activation, normal human peripheral blood neutrophil cells can be purified on a density gradient such as a Ficoll gradient. Other immune regulatory cells, such as T-lymphocytes and macrophages can be isolated by standard and known techniques. The neutrophils can be suspended in Dulbecco's phosphate-buffered saline supplemented with calcium and magnesium. One way to monitor neutrophil activation is to observe chemiluminescence. The neutrophils can be incubated for about fifteen minutes with the bryostatin. Then luminol (5-amino-2-3-dihydro-1, 4-phthalazinedione) can be added to a concentration of about 10 uM. Chemiluminescence can be measured in a luminometer as described in Allen et al, Biochemistry and Biophysics Research Communications, Vol. 69, pp. 245-522, 1976.

Alternatively, neutrophil activation can be assayed using a cytoxicity assay. Purified neutrophils can be incubated at a density of about $3\times10^{6}$ cells per ml with various concentrations of bryostatin. The cells can be washed and added to radiolabelled target cells. K562 tumor cells and [51] Cr-label can be used to measure cytoxicity. The target cells are preincubated with the radiolabelled substance to effect uptake of the radiolabel. The target cells can be mixed with the neutrophils at various effector to target cell ratios, such as between about 50:1 and 5:1, Release of label from the target cells can be measured either by assaying the supernatant or the cells after centrifugation. Of course, other methods known in the art, such as using other assays of immune regulatory cell cytotoxicity can be used. Bryostatin 1 has activity toward neutrophils at about $10^{-10}$ to $10^{-7}$M, with a maximum activation effect on neutrophil cytotoxicity of K562 target cells at a concentration of about $10^{-7}$M.

It may be advantageous to treat either cells or mammals concommitantly with bryostatin and a chemotherapeutic agent or ionizing radiation. For example, it may be desirable to purge a cell suspension of all malignant cells while sustaining the viability and proliferation of hematopoietic progenitor cells. This can be done using chemical agents known in the art, such as adriamycin, cytosine arabinoside, vincristine, cyclophosphamide, busulfan, prednisone, and M-AMSA. Purging can also be accomplished with tumor specific antibodies. Many such antibodies are known in the art. Alternatively, chemotherapeutic agents or anti-tumor antibodies can be administered directly to the mammal which is receiving bryostatin. Bryostatin may enhance the antineoplastic activity of the chemotherapeutic agent or antibody. Alternatively, use of bryostatin should allow higher amounts of the antineoplastic agent to be used, because of a reduction in the deleterious effects upon hematopoiesis.

The bryostatins may be used to counteract the effects of many types of diseases. For example, anemias in which erythroid cell populations are low often require transfusions. Use of bryostatins to stimulate erythroid cell formation could prevent the need for transfusions. Such anemias include anemia of chronic disease, congenital anemias, acquired aplastic anemia, acquired or congenital pancytopenia, and Fanconi's anemia. Similarly after or during prolonged chemotherapy, hematopoietic cells are often depleted. Thus, bryostatins could serve to bolster such cell populations. In addition, in patients who are compromised in their immune systems, either due to immunosuppressive drugs or immunosuppressive diseases, the bryostatins can be used to stimulate the patients' white cells to fight infections (by activation of neutrophils). Other situations where bryostatins can be advantageously used are in situations where populations of humans and other mammals have been exposed to radiation, such as from nuclear weapons or from environmental leakage from nuclear power stations. In those cases, administration of bryostatins could relieve the deleterious and often fatal effects of radiation on hematopoietic cells.

The bryostatins can be administered by any means known in the art. Because the desired targets of the bryostatins occur primarily in the bone marrow and blood system it is desirable that the method of administration allows the bryostatins to reach those tissues. Bryostatins can be administered parenterally by injection, rapid infusion, nasopharyngeal adsorption, dermal adsorption, or orally. One preferred method of administration of bryostatins is continuous infusion. For example an osmotic micropump can be implanted subcutaneously and bryostatin infused over a period of days, Generally between 10 and 1000 ug per kilogram per day is desirable to stimulate hematopoietic cells.

The following examples are meant to illustrate the foregoing invention and do not limit the invention, which is specifically defined by the claims appended below.

## EXAMPLES

Example 1

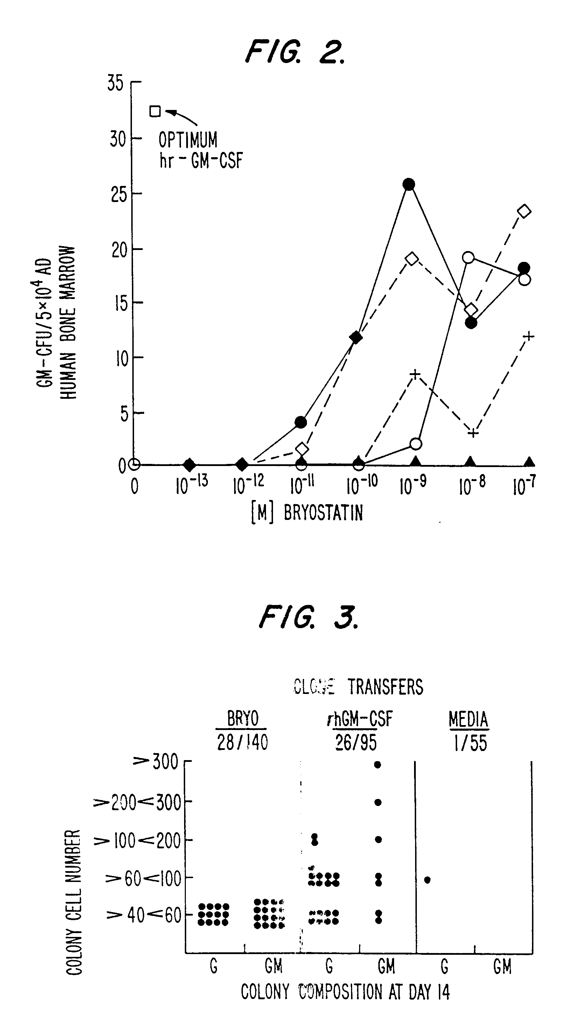

This example demonstrates the stimulatory effect of bryostatins on granulocyte-macrophage colony formation. Granulocyte-macrophage colony-forming unit (CFU-GM) colonies were enumerated from light-density, adherence-depleted normal human bone marrow cells. Cells were suspended in supplemented McCoy's 5A medium containing 0.3% agar and plated in 35-mm Petri dishes as described in Pike et al, J. Cell Physiol., Vol. 76, pp. 77-83, 1970. Results are shown in Figure 2. Colonies were enumerated following incubation of bone marrow with cells with rHGM-CSF at 50 ng/ml (Genetics Institute) or bryostatin 1 (●) bryostatin 3(o), bryostatin 8(♦), bryostatin 9(+), or bryostatin 13 (▲) at the concentrations indicated. After 14 days of culture at 37°C in a humidified atmosphere containing 7.5% $CO_2$, colonies of greater or equal to 50 cells were scored. Representative results from one of several experiments are reported as the mean colony number from quadruplicate samples. SEM is within 10% of the mean number.

Bryostatins 1, 3, 8, and 9 were found to mediate a dose-dependent stimulation of CFU-GM with the maximal effective concentration between 1 and 100 nM. Colony stimulating activity was observed with a concentration as low as 0.01 nM for bryostatins 1 and 8, which are the most potent of the four active congeners tested. While the amplitude of granulocyte-macrophage colony response was similar for bryostatin 3 at the higher concentrations (i.e., 10-100 nM), this agent was clearly less potent than bryostatin 1 or 8. Bryostatin 9 was only a weak stimulator of normal colony growth, whereas bryostatin 13 was completely inactive.

Analysis of colony morphology revealed that bryostatin 1, like rHGM-CSF, stimulated primarily mixed granulocyte-macrophage and pure granulocyte colonies at 7 and 14 days of culture.

Example 2

This example shows that bryostatin 1 exerts its effect directly on cells.

Assays were performed essentially as described in Metcalf et al, Blood, Vol. 67, pp. 37-45, 1986. Briefly, cultures of normal human bone marrow were initiated in medium containing 10 nM bryostatin 1. Following 4 days of growth, clones of 4-8 cells were carefully removed using a glass pipette to avoid contamination by surrounding cells and transferred to secondary plates containing either rhGM-CSF (50 ng/ml) or bryostatin 1 (10 nM) as the source of CSF. After an additional 10 days of culture, the number of clones that had proliferated to form a colony of 40 cells or more was determined. Further growth was not observed in colonies transferred to plates containing no added CSF. (See Figure 3.)

Each symbol (●) represents a colony. Its height in each column is proportional to the number of cells in that colony. The data directly above sections G of the column represent colonies composed entirely of granulocytic cells. The data above sections GM in the column represent colonies composed of both granulocytic and monocytic cells. No colonies in secondary plates were detected that were composed of macrophage-monocyte cells only. The numerator in the fraction at the top of each column represents the number of colonies that developed in the secondary plate, and the denominator represents the total number of clones transferred. The clones from primary plates initiated with bryostatin produced full-size colonies only on the secondary plates to which bryostatin or rhGM-CSF had been added. This indicates that the stimulatory effect of bryostatin 1 and rhGM-CSF is exerted directly.

Further, an attempt to produce a conditioned medium from bone marrow cells in the presence of bryostatin was unsuccessful. Nonadherence-depleted human bone marrow, which was composed of a mixture of progenitor plus ancillary cells, was incubated in culture medium that contained a maximal stimulatory concentration of bryostatin (i.e. 1-10 nM) for 5 days. The supernatant was aspirated and tested for the presences of a GM-CSF after fractionation by passage over a Sephadex G-25 gel filtration column to remove the bryostatin (i.e., $M_r$, 884). The resulting void volume, which contains the large molecular weight polypeptide growth factors, was evaluated for CSF activity. This supernatant was tested at both 10% and 50% (vol/vol) final concentrations in the culture plates and failed to stimulate GM-CFU growth.

Example 3

This example demonstrates the stimulatory effect of bryostatin 1 on human erythrocyte progenitors.

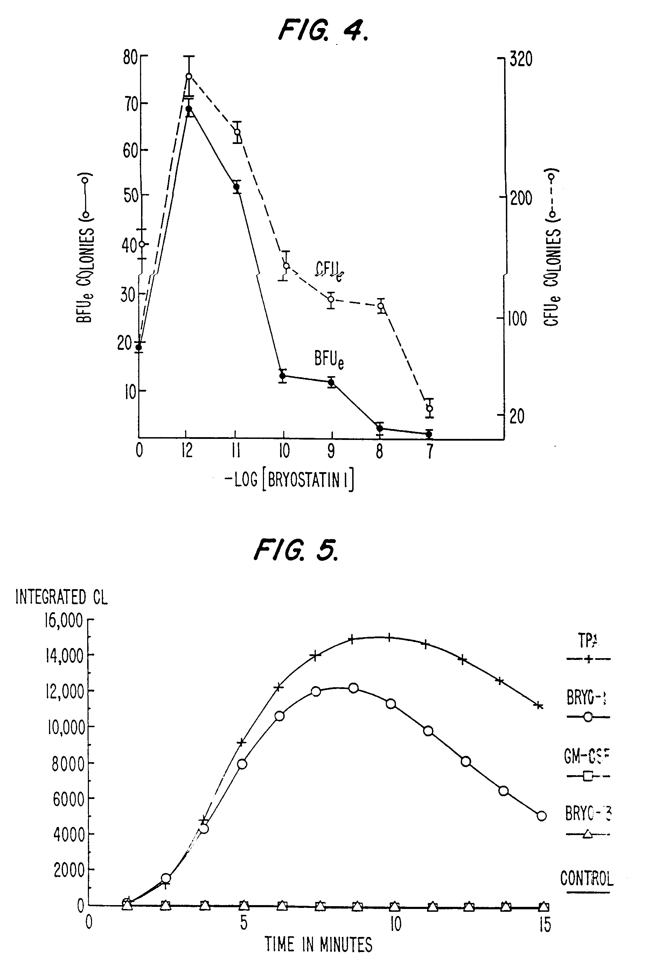

Assays for both early (BFU-E) and late (CFU-E) progenitors were enumerated as described in McLeod et al, Blood, Vol. 44, p. 517 (1974) using the plasma-clot method and optimal concentrations of erythropoietin. Bryostatin 1 stimulated both early BFU-E and late CFU-E human erythroid progenitors growth in plasma clots. See Figure 4. The maximal stimulatory concentration was between 1 and 10 pM, with an inhibitory effect noted at concentrations above 1 nM.

Example 4

This example demonstrates that bryostatin 1 but not 13 causes neutrophils to chemiluminesce in the presence of luminol. In addition neutrophils are shown to be activated to kill K562 target cells by bryostatin 1.

Normal human peripheral blood neutrophils were highly purified on a double Ficoll density gradient and suspended in Dulbecco's phosphate-buffered saline supplemented with $Ca^{2+}$ and $Mg^{2+}$. First, substances to be tested were incubated with the neutrophils for 15 min. At this point 10 uM luminol (5-amino-2, 3-dihydro-1, 4-phtyalazinedione) was added to each sample. The generation of chemiluminescence was measured in a luminometer at 1.25 min intervals for up to 20 min.

Bryostatin 1, but not bryostatin 13, was found to be a potent activator of neutrophil chemiluminescence, a measure of oxide radical formation. Activation follows a similar time course to that for the known activator phorbol 12-myristate 13-acetate. See Figure 5. TPA was at 150 nM and recombinant human GM-CSF at 50 ng/ml.

Furthermore, activation of neutrophil cytotoxicity, as measured by killing of $^{51}$Cr-labeled K562 tumor target cells was also sitmulated by bryostatin 1 (Table 1). Neutrophils were purified as described above. Neutrophils were incubated at a density of $3 \times 10^6$ cells per ml first with various concentrations of bryostatin 1 (0.1 uM to 0.1 nM), rhGM-CSF (50 ng/ml), or in medium alone for 2 hr. Following two washes, the cells were added to the $^{51}$Cr-labeled K562 target cells at effector/target cell ratios of 30:1, 15:1, or 7.5:1 as indicated. Control plates containing $^{51}$Cr-labeled K562 cells and either bryostatin or medium alone were evaluated to determine the spontaneous release of isotope. The plates containing the cells were centrifuged at 250 x g for 2 min and then incubated for 3 hrs at 37°C. One hundred microliters of the supernatant was harvested from each well, and the amount of radioactivity present was determined in a gamma scintillation counter. The maximum release of $^{51}$Cr was determined by assaying supernatant obtained from wells in which the cells were completely lysed with 6 M HCl.

Neutrophil activation was maximal between 10 and 100 nM bryostatin 1, a concentration range that can promote myeloid hematopoietic growth. Whereas rhGM-CSF has been reported to activate eosinophils and neutrophils we were unable to detect either the induction of chemiluminescence or of cytotoxicity in neutrophils when rhGM-CSF at 50 ng/ml, a maximal-stimulatory concentration for granulocyte-macrophage-colony formation.

Example 5

This example demonstrates that bryostatin 1 is not toxic to a mammal and stimulates multipotential hematopoietic progenitors in vivo.

Using osmotic micropumps (Alzet) implanted subcutaneously in 6 week old female BDF$_1$ mice, bryostatin infused for 7 days had no major organ toxic effects at any of the doses employed. A dose-response stimulatory effect on multi-potential hematopoietic progenitors was noted. The administration of 144 ug/kg/d (max. dose) increased CFU-S, CFU-GM and BFU-E compared to control animals infused with vehicle alone. CFU-S from spleen cells of treated animals were increased 2-fold (19 vs. 39 colonies per $10^6$ cells injected), while bone marrow CFU-S was not altered. Likewise, CFU-GM colonies from spleen cells were increased 3.6-fold (52 vs. 188 per $10^6$ cells injected), and increased 2-fold (73 vs. 150 per $10^5$ nuc. cells) in bone marrow. Erythroid BFU-E colonies from spleen cells also demonstrated a 2-fold increase (28 vs. 58 per $10^6$ cells), while no change in bone marrow BFU-E progenitors was found. The increment in progenitor cells was not accompanied by a significant change in the differential peripheral white blood cell, hemocrit, bone marrow cellularity or spleen weights.

The results demonstrate that continuous infusion of bryostatin is non-toxic and can stimulate effectively murine multi-potential hematopoietic progenitor cells in vivo. The stimulatory effect of bryo is similar to its effect on multipotential progenitor cells in vitro and mimics the reported effect of infusion of rIL-3 and rGM-CSF.

## TABLE 1

### Activated neutrophil cytotoxicity for K562 target cells

| Addition | % specific lysis at effector/target ratio | | |
|---|---|---|---|
| | 30 | 15 | 7.5 |
| Neutrophils Plus | | | |
| Bryostatin 1 | | | |
| $10^{-7}$M | 102.8 | 18.1 | 7.8 |
| $10^{-8}$M | 8.4 | 2.4 | 0 |
| $10^{-9}$M | 0.9 | 1.8 | 0 |
| $10^{-10}$M | -0.8 | 1.0 | 0.9 |
| Medium alone | 0.9 | 1.0 | -0.8 |
| rHGM-CSF (50 ng/ml) | 1.6 | 0.9 | -0.7 |
| Bryostatin alone | | | |
| $10^{-7}$M | 1.5 | 0.3 | -0.6 |
| $10^{-8}$M | 0.9 | 1.0 | 0.3 |

Assessment of neutrophil cytotoxicity induced by bryostatin 1. K562 cells were labeled with $Na^{51}Cr_2O_4$ and adjusted to a concentration of $10^3$ cells per ml in RPMI 1640 medium. Activated neutrophil cytotoxicity was determined. The results are expressed as the percentage of specific isotope released at the indicated effector/target cell ratio calculated as follows: % specific lysis = [(mean cpm of sample - mean cpm spontaneously released)/(mean cpm maximal -mean cpm spontaneously released)] x 100. Maximum induced cpm released as 7364 ± 149. The maximum mean % specific lysis from triplicate determinations from a representative experiment is shown.

## Claims

1. A method of stimulating stem cells to form erythrocytes comprising: obtaining a cell preparation containing stem cells; and

   incubating the cell preparation in vitro with a bryostatin having an acylated C20 carbon in an amount effective to stimulate formation of erythrocytes, whereby said formation is stimulated.

2. The method of claim 1 wherein the stem cells are obtained from bone marrow.

3. The method of claim 1 wherein the stem cells are obtained from peripheral blood cells.

4. The method of claim 1 wherein the effective amount of bryostatin is a concentration of from about $10^{-12}$M to about $10^{-7}$M.

5. The method of claim 4 wherein the concentration of bryostatin is about $10^{-10}$M to about $10^{-12}$M and wherein erythropoietin is incubated with the cell preparation, and the cells formed are erythrocytes.

6. The method of claim 1 wherein a chemotherapeutic agent is also added to the cell preparation in an amount effective to kill malignant cells.

7. The method of claim 1 wherein a monoclonal antibody is used to remove malignant cells from the cell preparation.

8. The method of claim 6 wherein the chemotherapeutic agent is bryostatin 13.

9. The method of claim 6 wherein the chemotherapeutic agent is selected from the group consisting of: adriamycin, cytosine arabanoside, vincristine, cyclophosphamide, busulfan, prednisone, and M-AMSA.

10. The method of claim 1 wherein the bryostatin is selected from bryostatin 1, 3, 8 or 9.

11. The method of claim 1 wherein the cell preparation is treated with ionizing radiation in an amount effective to kill malignant cells.

12. The use of a bryostatin having an acylated C20 carbon in the preparation of a composition for the stimulation of formation of erythrocytes.

13. The use of bryostatin 13 and bryostatin 1 in the preparation of a composition for use in arresting the growth of a tumour and stimulating the formation of erythrocytes respectively.

14. The use of claim 12 or 13 in which the composition is suitable for administration by continuous infusion.

15. The use of claim 12 or 13 in which the composition is of suitable form for subcutaneous administration.

16. The use of claim 12 in which the bryostatin is selected from bryostatin 1, 3, 8 or 9.

17. A method of activating the cytotoxicity of neutrophils and other immune regulatory cells, comprising:
    obtaining a cell preparation containing neutrophils or other immune regulatory cells;
    and
    incubating the cell preparation in vitro with an amount of a bryostatin having an acylated C20 carbon effective to activate the neutrophils or other immune regulatory cells, whereby said cells' cytotoxicity is activated.

18. The method of claim 17 wherein the amount of bryostatin is a concentration between about $10^{-7}$ M and about $10^{-10}$ M.

19. The method of claim 17 wherein the bryostatin is selected from bryostatin 1, 3, 8 or 9.

20. The use of a bryostatin having an acylated C20 carbon in the preparation of a composition for the activation of neutrophil or other immune regulatory cell cytotoxicity.

21. A use according to claim 21 in which the bryostatin is selected from bryostatin 1, 3, 8 or 9.

**Patentansprüche**

1. Verfahren zur Anregung von Stammzellen zur Erzeugung von Erythrocyten, das die folgenden Schritte umfaßt:
   Erhalt einer Zellpräparation, die Stammzellen enthält und
   Inkubierung der Zellpräparation in vitro mit einem Bryostatin mit einem acylierten C20-Kohlenstoff in einer Menge, die wirksam ist, um die Erzeugung von Erythrocyten anzuregen, wodurch die Erzeugung angeregt wird.

2. Verfahren nach Anspruch 1, wobei man die Stammzellen aus Knochenmark erhält.

3. Verfahren nach Anspruch 1, wobei man die Stammzellen aus peripheren Blutzellen erhält.

4. Verfahren nach Anspruch 1, wobei die wirksame Bryostatinmenge eine Konzentration von etwa $10^{-12}$M bis etwa $10^{-7}$ M ist.

5. Verfahren nach Anspruch 4, wobei die Bryostatinkonzentration etwa $10^{-10}$ M bis etwa $10^{-12}$M beträgt und wobei man Erythropoietin mit der Zellpräparation inkubiert und die erzeugten Zellen Erythrocyten sind.

6. Verfahren nach Anspruch 1, wobei man ferner der Zellpräparation ein chemotherapeutisches Mittel in einer Menge zugibt, die wirksam ist, um maligne Zellen zu töten.

**7.** Verfahren nach Anspruch 1, wobei ein monoclonaler Antikörper verwendet wird, um maligne Zellen aus der Zellpräparation zu entfernen.

**8.** Verfahren nach Anspruch 6, wobei das chemotherapeutische Mittel Bryostatin 13 ist.

**9.** Verfahren nach Anspruch 6, wobei das chemotherapeutische Mittel aus der Gruppe ausgewählt ist, die aus Adriamycin, Cytosin-Arabanosid, Vincristin, Cyclophosphamid, Busulfan, Prednison und M-AMSA besteht.

**10.** Verfahren nach Anspruch 1, wobei man das Bryostatin aus Bryostatin 1, 3, 8 oder 9 auswählt.

**11.** Verfahren nach Anspruch 1, wobei man die Zellpräparation mit ionisierender Strahlung in einer Menge behandelt, die wirksam ist, um maligne Zellen zu töten.

**12.** Verwendung eines Bryostatins mit einem acylierten C20-Kohlenstoff in der Präparation einer Zusammensetzung für die Anregung zur Bildung von Erythrocyten.

**13.** Verwendung von Bryostatin 13 und Bryostatin 1 in der Präparation einer Zusammensetzung zur Verwendung bei der Wachstumshemmung eines Tumors bzw. der Anregung zur Erzeugung von Erythrocyten.

**14.** Verwendung von Anspruch 12 oder 13, bei dem die Zusammensetzung zur Verabreichung mittels einer kontinuierlichen Infusion geeignet ist.

**15.** Verwendung von Anspruch 12 oder 13, bei dem die Zusammensetzung eine zur subkutanen Verabreichung geeignete Form aufweist.

**16.** Verwendung des Anspruchs 12, bei der man das Bryostatin aus Bryostatin 1, 3, 8 oder 9 auswählt.

**17.** Verfahren zur Aktivierung der Cytotoxizität von neutrophilen und anderen Immunregulatorzellen, das die folgenden Schritte umfaßt:
Erhalt einer Zellpräparation, die Neutrophile oder andere Immunregulatorzellen enthält und
Inkubierung der Zellpräparation in vitro mit einer Menge eines Bryostatins mit einem acylierten C20-Kohlenstoff, die wirksam ist, um die Neutrophile oder andere Immunregulatorzellen zu aktivieren, wodurch die Cytotoxizität der Zellen aktiviert wird.

**18.** Verfahren nach Anspruch 17, wobei die Bryostatinmenge eine Konzentration zwischen etwa $10^{-7}$ M und etwa $10^{-10}$ M beträgt.

**19.** Verfahren nach Anspruch 17, wobei man das Bryostatin aus Bryostatin 1, 3, 8 oder 9 auswählt.

**20.** Verwendung eines Bryostatins mit einem acylierten C20-Kohlenstoff bei der Präparation einer Zusammensetzung zur Aktivierung der Cytotoxizität von Neutrophilen oder anderen Immunregulatorzellen.

**21.** Verwendung nach Anspruch 20, wobei man das Bryostatin aus Bryostatin 1, 3, 8 oder 9 auswählt.

**Revendications**

**1.** Procédé de stimulation de cellules souches pour former des érythrocytes, comprenant les étapes qui consistent à :
obtenir une préparation cellulaire contenant des cellules souches; et
incuber la préparation cellulaire in vitro avec une bryostatine possédant un carbone C20 acylé, en une quantité efficace pour stimuler la formation d'érythrocytes, ce qui stimule ladite formation.

**2.** Procédé selon la revendication 1, dans lequel les cellules souches s'obtiennent à partir de moelle osseuse.

**3.** Procédé selon la revendication 1, dans lequel les cellules souches s'obtiennent à partir de globules sanguins périphériques.

**4.** Procédé selon la revendication 1, dans lequel la quantité efficace de bryostatine est une concentration d'environ $10^{-12}$M à $10^{-7}$M.

**5.** Procédé selon la revendication 4, dans lequel la concentration de bryostatine est d'environ $10^{-10}$M à environ $10^{-12}$M et dans lequel de l'érythropoïétine est incubée avec la préparation cellulaire, et les cellules formées sont des érythrocytes.

**6.** Procédé selon la revendication 1, dans lequel un agent chimiothérapique est aussi ajouté à la préparation cellulaire, en une quantité efficace pour tuer les cellules malignes.

**7.** Procédé selon la revendication 1, dans lequel un anticorps monoclonal est employé pour éliminer les cellules malignes de la préparation cellulaire.

**8.** Procédé selon la revendication 6, dans lequel l'agent chimiothérapique est la bryostatine 13.

**9.** Procédé selon la revendication 6, dans lequel l'agent chimiothérapique est choisi dans le groupe constitué par : l'adriamycine, la cytosine arabanoside, la vincristine, le cyclophosphamide, le busulfane, la prednisone et le M-AMSA.

**10.** Procédé selon la revendication 1, dans lequel la bryostatine est choisie parmi la bryostatine 1, 3, 8 ou 9.

**11.** Procédé selon la revendication 1, dans lequel la préparation cellulaire est traitée par un rayonnement ionisant dont la quantité est efficace pour tuer les cellules malignes.

**12.** Utilisation d'une bryostatine possédant un carbone C20 acylé dans la préparation d'une composition pour la stimulation de la formation d'érythrocytes.

**13.** Utilisation de bryostatine 13 et de bryostatine 1 dans la préparation de compositions utilisables respectivement pour stopper la croissance d'une tumeur et pour stimuler la formation d'érythrocytes.

**14.** Utilisation selon la revendication 12 ou 13, dans laquelle la composition convient à une administration par perfusion continue.

**15.** Utilisation selon la revendication 12 ou 13, dans laquelle la composition convient à une administration sous-cutanée.

**16.** Utilisation selon la revendication 12, dans laquelle la bryostatine est choisie parmi la bryostatine 1, 3, 8 ou 9.

**17.** Procédé d'activation de la cytotoxicité des neutrophiles et d'autres cellules immuno-régulatrices, comprenant les étapes qui consistent à :

obtenir une préparation cellulaire contenant des neutrophiles ou d'autres cellules immuno-régulatrices; et

incuber la préparation cellulaire in vitro avec une quantité de bryostatine possédant un carbone C20 acylé efficace pour activer les neutrophiles ou les autres cellules immuno-régulatrices, ce qui active ladite cytotoxicité des cellules.

**18.** Procédé selon la revendication 17, dans lequel la quantité de bryostatine est une concentration d'environ $10^{-7}$M à $10^{-10}$M.

**19.** Procédé selon la revendication 17, dans lequel la bryostatine est choisie parmi la bryostatine 1, 3, 8 ou 9.

**20.** Utilisation d'une bryostatine possédant un carbone C20 acylé dans la préparation d'une composition pour l'activation de la cytotoxicité des neutrophiles ou d'autres cellules immuno-régulatrices.

**21.** Utilisation selon la revendication 20, dans laquelle la bryostatine est choisie parmi la bryostatine 1, 3, 8 ou 9.

# FIG. 1.

A — BRYOSTATIN 1

B — BRYOSTATIN 9

C — BRYOSTATIN 8

D — BRYOSTATIN 3

E — BRYOSTATIN 13

# FIG. 2.

# FIG. 3.

# FIG. 4.

# FIG. 5.